# FASCICULE DE BREVET EUROPEEN

(11) **EP 0 599 752 B1**
(45) Date de publication et mention de la délivrance du brevet: **23.04.1997**
(21) Numéro de dépôt: 93420465.2
(22) Date de dépôt: 23.11.1993
(51) Int. Cl.: A61B 17/74

(54) **Dispositif d'ostéosynthèse pour fractures trochantérienne ou trochantéro-diaphysaire**
Osteosynthesevorrichtung für trochantäre oder intertrochantäre Frakturen
Osteosynthesis device for trochanteric or intertrochanteric fractures

(30) Priorité: 24.11.1992 FR 9214111
(43) Date de publication de la demande: 01.06.1994
(73) Titulaire: FIXANO SA, F-01000 Bourg en Bresse (FR)
(72) Inventeur: De la Caffinière, Jean-Yves, F-75006 Paris (FR)
(74) Mandataire: Maureau, Philippe

(56) Documents cités:
- EP-A- 0 441 577
- DE-U- 9 206 580
- FR-A- 2 674 119
- US-A- 4 827 917

## Description

La présente invention concerne un dispositif d'ostéosynthèse pour fracture trochantérienne ou trochantéro-diaphysaire.

La réduction d'une telle fracture et le maintien des parties de l'os fracturé le temps que s'opère la consolidation osseuse impliquent la mise en place d'au moins une vis à l'intérieur du col du fémur, venant prendre appui, d'une part, dans la tête fémorale et, d'autre part, au niveau de la corticale osseuse.

Pour ce type de fracture spécifique, dont la réparation nécessite un appui diaphysaire solide, il est connu par le brevet US N° 4 827 917 d'utiliser un clou médullaire, qui est verrouillé en partie distale et qui comprend, en partie proximale, au moins un trou aménagé selon un axe sensiblement parallèle à l'axe du col du fémur, la vis précitée étant destinée à être engagée au travers de ce trou et à être immobilisée par rapport au clou.

De préférence, des vis autoforeuses et autotaraudeuses sont employées, ces vis présentant une extrémité distale filetée de diamètre supérieur à celui de leur corps. Dans ce cas, comme le montrent la demande de brevet européen N° 0 441 577 ou le modèle d'utilité allemand N° 92 06 580, le trou précité est aménagé dans le clou au diamètre de l'extrémité distale filetée de la vis et, après mise en place de cette dernière, un manchon est engagé sur l'extrémité proximale de la vis et au travers du trou que comprend le clou, pour remplir l'espace entre le corps de la vis et le clou.

Le clou, généralement tubulaire, est taraudé à son extrémité proximale, ce qui permet la mise en place d'une vis de pression venant immobiliser la vis engagée dans l'os ou le manchon engagé sur la vis, par rapport au clou.

Ce moyen d'immobilisation présente l'inconvénient d'être complexe à utiliser puisqu'il faut venir visser et serrer cette vis de pression à l'extrémité proximale du clou tout en maintenant le clou et la vis dans leur position relative adéquate.

De plus, compte tenu de l'angle que forment la vis et le clou, la surface d'appui de l'extrémité de la vis de pression contre la vis engagée dans l'os ou le manchon précité est limitée, de sorte qu'une possibilité de jeu entre le clou et la vis ou le manchon peut se produire dans le temps, sous l'effet des contraintes répétées qui s'exercent à cet endroit précis de l'os. Un couple de serrage important est nécessaire pour limiter au maximum ce risque de jeu, ce qui contribue à rendre complexe la pose de ces dispositifs.

Enfin, une telle vis de pression rend complexe la fabrication de ces dispositifs.

Par ailleurs, dans les dispositifs antérieurs, la ou les vis sont immobilisées de manière rigide par rapport au clou. Ce montage rigide ne s'avère guère souhaitable pour la parfaite consolidation de l'os, compte tenu de ce que le positionnement des parties de l'os fracturé peut évoluer au cours de la consolidation osseuse. Dans certains cas extrêmes, l'extrémité distale des vis engagées dans l'os a pu traverser la tête fémorale au cours de la consolidation osseuse.

L'invention vise à remédier à ces différents inconvénients.

Le dispositif auquel elle s'applique comprend un clou médullaire qui est verrouillé à l'os en partie distale et qui comprend, en partie proximale, au moins un trou aménagé selon un axe sensiblement parallèle à l'axe du col du fémur, au moins une vis autoforeuse et autotaraudeuse destinée à être engagée dans l'os au travers du trou, au moins un manchon destiné à être engagé, après mise en place de la vis, sur l'extrémité proximale de la vis et au travers du trou que comprend le clou, pour remplir l'espace existant entre le corps de la vis et le clou, et des moyens pour immobiliser le manchon par rapport au clou, le corps de la ou des vis présentant une section transversale circulaire.

Selon l'invention, le ou les trous du clou sont taraudés, et chaque manchon comprend un filetage faisant saillie de sa paroi extérieure, ce filetage correspondant au taraudage du ou des trous du clou, un épaulement côté proximal venant en appui contre le clou en fin de vissage du manchon dans le trou et une extrémité proximale profilée pour permettre le vissage du manchon au travers du trou que comprend le clou.

Après mise en place du clou et de la vis, le manchon est assemblé au clou par vissage et serrage. Ce vissage et serrage s'opère par le même trou que celui ayant permis l'introduction des vis dans l'os.

Il n'est donc plus nécessaire, comme selon la technique antérieure, de mettre en place une vis de pression, ni de maintenir la vis et le manchon en position relative adéquate, le temps que cette vis de pression soit serrée.

En outre, l'immobilisation du manchon par rapport au clou est beaucoup plus fiable dans le temps qu'avec les dispositifs de la technique antérieure et la fabrication du dispositif est nettement simplifiée.

Avantageusement, le manchon présente une longueur telle que son alésage débouche sur l'extérieur de l'os après mise en place du manchon sur le clou, et cet alésage est tel qu'il permet un coulissement de la vis par rapport au manchon.

La position de la vis par rapport au clou peut ainsi évoluer et s'adapter à l'évolution de la position des parties de l'os au cours de la consolidation osseuse.

Avantageusement, le manchon présente un prolongement côté distal, de manière à faire saillie au-delà du clou après mise en place, permettant ainsi d'assurer un parfait guidage axial de la vis en coulissement et d'éliminer tout risque de flexion de celle-ci sous l'effet des contraintes verticales exercées sur l'os.

Pour sa bonne compréhension, l'invention va être à nouveau décrite ci-dessous, en référence au dessin schématique annexé représentant, à titre d'exemple non limitatif, une forme de réalisation préférée du dispositif d'ostéosynthèse pour fracture trochantérienne ou trochantéro-diaphysaire qu'elle concerne.
La figure 1 en est une vue en perspective et en coupe longitudinale, après mise en place dans un os et
la Figure 2 est une vue en perspective et à échelle agrandie d'un élément qui le compose.

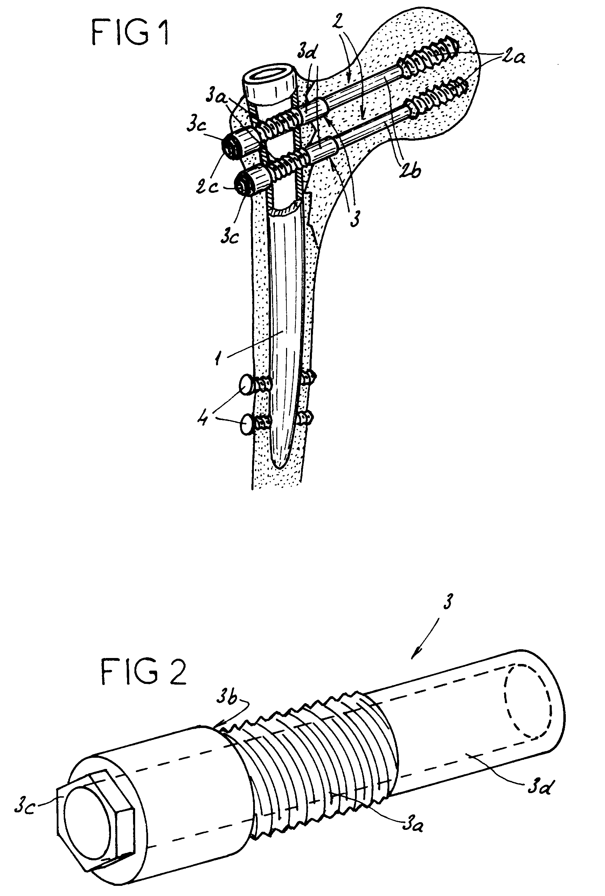

La figure 1 représente un dispositif d'ostéosynthèse pour fracture trochantérienne ou trochantéro-diaphysaire.

Ce dispositif comprend un clou médullaire 1, deux vis autoforeuses et autotaraudeuses 2 et deux manchons 3.

Le clou 1 est destiné à être verrouillé à l'os en partie distale par deux vis transversales 4 et comprend, en partie proximale, deux trous taraudés, aménagés selon un axe sensiblement parallèle à l'axe du col du fémur, pour le passage des vis 2.

Ces dernières sont de section transversale circulaire et présentent une extrémité distale filetée 2a dont le diamètre est supérieur à celui de leur corps 2b. Les trous taraudés précités sont aménagés dans le clou 1 au diamètre extérieur de ces extrémités 2a, pour permettre le passage de celles-ci au travers d'eux.

Après mise en place du clou 1 dans le canal médullaire de l'os, les vis 2 sont à leur tour mises en place dans l'os, jusqu'à prendre appui en partie distale dans la tête du fémur. Les deux manchons 3 sont ensuite engagés sur l'extrémité proximale 2c des vis 2 pour remplir l'espace entre le corps 2b des vis 2 et le clou 1.

La figure 2 montre plus particulièrement l'un de ces deux manchons 3.

Il comprend :
- un filetage médian 3a faisant saillie de sa paroi extérieure, ce filetage 3a correspondant au taraudage des trous du clou 1 ;
- un épaulement 3b côté proximal, destiné à venir en appui contre le clou 1 en fin de vissage du manchon 3 dans le trou taraudé ;
- une extrémité proximale 3c présentant un profil hexagonal pour permettre le vissage du manchon 3 au travers du trou que comprend le clou 1 et
- un prolongement 3d du côté distal.

En outre, le manchon 3 présente une longueur telle que son alésage débouche sur l'extérieur de l'os après mise en place du manchon, ainsi que le montre la figure 1, et cet alésage est tel qu'il permet le coulissement de la vis 2 par rapport au manchon 3.

Ainsi, selon l'invention, les manchons 3 sont assemblés au clou 1 par vissage et serrage après mise en place du clou 1 et des vis 2 dans l'os. Ce vissage et serrage s'opère par les mêmes trous que ceux ayant permis l'introduction des vis 2 dans l'os.

L'immobilisation des manchons 3 par rapport au clou 1 est très fiable dans le temps et résiste parfaitement aux contraintes répétées qui s'exercent à cet endroit précis de l'os. Aucune possibilité de jeu entre le clou 1 et les vis 2 ou les manchons 3 n'existe.

De plus, grâce au fait que les alésages des manchons 3 débouchent sur l'extérieur et que ces alésages permettent un coulissement des vis 2 par rapport aux manchons 3, la position des vis 2 par rapport au clou 1 peut évoluer dans le temps et s'adapter à l'évolution de la position des parties de l'os au cours de la consolidation osseuse.

Une telle ostéosynthèse dynamique permet l'obtention d'une parfaite consolidation.

En outre, ainsi que cela apparaît à la figure 1, les prolongements 3d des manchons 3 font saillie au-delà du clou 1 après mise en place, ce qui permet d'assurer un parfait guidage axial des vis 2 en coulissement et d'éviter tout risque de flexion de celles-ci sous l'effet des contraintes verticales exercées sur l'os ou de l'évolution de la position relative des parties de l'os fracturé au cours de la consolidation.

Il va de soi que l'invention n'est pas limitée à la forme de réalisation préférée qui vient d'être décrite mais qu'elle en embrasse au contraire toutes les variantes de réalisation.

Ainsi, l'on ne s'écarterait pas de l'invention en ne prévoyant qu'une seule vis 2, et donc qu'un seul manchon 3.

## Revendications

1. Dispositif d'ostéosynthèse pour fracture trochantérienne ou trochantéro-diaphysaire, comprenant un clou médullaire (1) qui est verrouillé à l'os en partie distale et qui comprend, en partie proximale, au moins un trou aménagé selon un axe sensiblement parallèle à l'axe du col du fémur, au moins une vis autoforeuse et autotaraudeuse (2) destinée à être engagée dans l'os au travers du trou, au moins un manchon (3) destiné à être engagé, après mise en place de la vis (2), sur l'extrémité proximale (2c) de la vis (2) et au travers du trou que comprend le clou (1), pour remplir l'espace existant entre le corps (2b) de la vis (2) et le clou (1), et des moyens pour immobiliser le manchon (3) par rapport au clou (1), le corps de la ou des vis (2) présentant une section transversale circulaire, caractérisé en ce que le ou les trous du clou (1) sont taraudés, et en ce que chaque manchon (3) comprend un filetage (3a) faisant saillie de sa paroi extérieure, ce filetage (3a) correspondant au taraudage du ou des trous du clou (1), un épaulement (3b) côté proximal venant en appui contre le clou (1) en fin de vissage du manchon (3) dans le trou et une extrémité proximale (3c) profilée pour permettre le vissage du manchon (3) au travers du trou que comprend le clou (1).

2. Dispositif selon la revendication 1, caractérisé en ce que chaque manchon (3) présente une longueur telle que son alésage débouche sur l'extérieur de l'os après mise en place du manchon (3) sur le clou (1), et en ce que cet alésage est tel qu'il permet le coulissement de la vis (2) par rapport au manchon (3).

3. Dispositif selon la revendication 1 ou la revendication 2, caractérisé en ce que chaque manchon (3) présente un prolongement (3d) côté distal, de manière à faire saillie au-delà du clou (1) après mise en place, permettant ainsi d'assurer un parfait guidage axial de la vis (2) en coulissement et d'éliminer tout risque de flexion de celle-ci sous l'effet des contraintes verticales exercées sur l'os.

## Claims

1. Osteosynthesis device for trochanteric or trochantero-diaphyseal fracture, comprising a medullary nail (1) which is locked to the distal part of the bone and which comprises, in the proximal part, at least one hole formed along an axis substantially parallel to the axis of the neck of the femur, at least one self-drilling and self-tapping screw (2) designed to be engaged in the bone through a hole, at least one sleeve (3) designed to be engaged, after the screw (2) has been put in place, on the proximal end (2c) of the screw (2) and through the hole which the nail (1) comprises, so as to fill the space which exists between the body (2b) of the screw (2) and the nail (1), and means of immobilising the sleeve (3) with respect to the nail (1), the body of the screw or screws (2) having a circular cross section, characterised in that the hole or holes in the nail (1) are threaded, and in that each sleeve (3) comprises a thread (3a) projecting from its external wall, this thread (3a) corresponding to the thread of the hole or holes in the nail (1), a shoulder (3b) on the proximal side bearing against the nail (1) once the screwing of the sleeve (3) into the hole is complete, and a profiled proximal end (3c) to enable the sleeve (3) to be screwed through the hole which the nail (1) comprises.

2. Device according to Claim 1, characterised in that the length of each sleeve (3) is such that its bore opens out outside the bone once the sleeve (3) has been put in place on the nail (1), and in that this bore is such that it enables the screw (2) to slide with respect to the sleeve (3).

3. Device according to Claim 1 or Claim 2, characterised in that each sleeve (3) has an extension (3d) on the distal side, so that it projects beyond the nail (1) following the putting in place, thereby affording perfect axial sliding guidance of the screw (2) and eliminating any risk of flexion thereof under the effect of the vertical forces exerted on the bone.

## Patentansprüche

1. Osteosynthesevorrichtung für eine trochantäre oder intertrochantäre Fraktur, mit einem medullaren Nagel (1), der am distalen Teil am Knochen verriegelt ist und der am proximalen Teil mindestens ein Loch, das entlang einer im wesentlichen parallel zur Achse des Oberschenkelhalses verlaufenden Achse eingearbeitet ist, mindestens eine selbstborende und selbstschneidende Schraube (2), die dazu dient, um durch das Loch in den Knochen eingeführt zu werden, mindestens einen Stutzen (3), der nach dem Anbringen der Schraube (2) an dem proximalen Ende (2c) der Schraube (2) und durch das Loch, das der Nagel (1) aufweist, eingeführt werden muß, um den zwischen dem Körper (2b) der Schraube (2) und dem Nagel (1) entstehenden Raum auszufüllen, sowie Mittel zum Festelegen der Muffe (3) bezüglich des Nagels (1) aufweist, wobei der Körper der Schraube oder der Schrauben (2) einen kreisförmigen Querschnitt hat,
**dadurch gekennzeichnet,** daß das oder die Löcher des Nagels (1) mit Innengewinde versehen sind und daß jeder Stutzen (3) ein Außengewinde (3a) aufweist, das von seiner Außenwand hervorsteht, wobei das Außengewinde (3a) dem Innengewinde des oder der Löcher des Nagels (1) entspricht und eine Schulter (3b) auf der proximalen Seite am Ende des Einschraubens des Stutzens (3) in das Loch gegen den Nagel (1) anschlägt, und mit einem mit einem Profil versehenen proximalen Ende (3c), um das Einschrauben des Stutzens durch das Loch, das der Nagel (1) aufweist, zu ermöglichen.

2. Vorrichtung nach Anspruch 1,
**dadurch gekennzeichnet,** daß jeder Stutzen (3) eine derartige Länge aufweist, daß seine Bohrung nach dem Einsetzen des Stutzens (3) auf den Nagel (1) zur Außenseite des Knochens hin offen ist, und daß diese Bohrung so ist, daß sie ein Gleiten der Schraube (2) bezüglich des Stutzens (3) ermöglicht.

3. Vorrichtung nach Anspruch 1 oder 2,
**dadurch gekennzeichnet,** daß jeder Stutzen (3) eine Verlängerung (3d) auf der distalen Seite aufweist, so daß er nach dem Einsetzen über den Nagel (1) hinausragt, wodurch eine vollkommene axiale Gleitführung der Schraube (2) ermöglicht wird und jegliche Gefahr ihrer Biegung unter der Einwirkung auf den Knochen ausgeübter vertikaler Spannungen eliminiert wird.
